# FASCICULE DE BREVET EUROPEEN

(11) **EP 0 692 480 B1**
(45) Date de publication et mention de la délivrance du brevet: **25.10.2000**
(21) Numéro de dépôt: 95401611.9
(22) Date de dépôt: 05.07.1995
(51) Int. Cl.: C07D 311/62

(54) **Procédé de préparation d'extraits contenant des composés polyphénoliques oligomères de type catéchiques à partir de sources végétales et extraits obtenus**
Verfahren zur Herstellung von Extrakten enthaltende polyphenalische oligomere Verbindungen von Catechin-Typ, aus pflanzenartigen Quellen, und diese erhaltene Extrakte
Process for the preparation of extracts containing polyphenolic oligomer compounds of catechin type from plant extracts and the so obtained extracts

(30) Priorité: 12.07.1994 FR 9408615
(43) Date de publication de la demande: 17.01.1996
(73) Titulaire: BERKEM, 24680 Gardonne (FR)
(72) Inventeur: Nkiliza, Jean, F-33220 Port Sainte Foy (FR); Marzelle, Jean-Claude, F-24680 Gardonne (FR)
(74) Mandataire: Peuscet, Jacques

(56) Documents cités:
- US-A- 3 436 407

## Description

La présente invention concerne un procédé de préparation d'extraits contenant des composés polyphénoliques oligomères de type catéchique ou oligomères flavanoliques à partir de sources végétales variées et les extraits destinés à l'industrie pharmaceutique, cosmétique et alimentaire obtenus.

Les polyphénols catéchiques ou tanins condensés, souvent appelés proanthocyanidols, sont des substances largement répandues dans les végétaux, notamment les pépins de raisin, l'écorce de pin et le thé vert. L'élément structural de base de ces polymères est une flavan-3-ol, dans laquelle le schéma d'hydroxylation des noyaux aromatiques varie aussi bien dans une même plante que d'une plante à l'autre.

Ces polyphénols sont généralement présents avec des degrés de polymérisation différents allant du monomère, des oligomères (dimère, trimère), jusqu'à des polymères de haut poids moléculaire appelés tanins.

Parmi ces polyphénols de type catéchique à structure très diversifiée, seuls les oligomères possèdent des propriétés biologiques intéressantes. A titre indicatif, on peut citer parmi les propriétés reconnues pour ces oligomères : l'augmentation de la résistance et la diminution de la perméabilité des capillaires sanguins, l'effet capteur des radicaux libres, l'action antimicrobienne et antivirale, l'action anti-inflammatoire, l'action comme filtre solaire et l'action antioxydante. En revanche, les hauts polymères sont dépourvus d'intérêt pharmacologique et doivent, par conséquent, être éliminés lors du processus d'extraction d'une source végétale.

De nombreux procédés de préparation d'extraits d'oligomères de phénols de type catéchique sont connus. On peut citer, à titre d'exemple, les brevets suivants : FR-A-1 427 100, FR-A-2 092 743, FR-A-2 372 823, FR-A-2 643 073, US-A-4 698 360, EP-A-348 781. Mais les extraits d'oligomères obtenus par ces procédés contiennent souvent des taux non négligeables de monomères. Or, il est bien connu que les monomères sont moins actifs que les dimères et les trimères correspondants. La proportion de monomère et la nature des oligomères peuvent varier selon la nature de la plante ou la partie traitée, les conditions de culture et le procédé d'extraction. Il est donc intéressant, dans certains cas, de réduire la teneur en monomères, voire de la supprimer.

EP-A-348 781 décrit un procédé de traitement d'un extrait qui, appliqué à l'extrait de pépin de raisin, permet d'obtenir des oligomères ayant une teneur en monomères inférieure à 1 %. Ce procédé met en oeuvre, soit une ultrafiltration d'un concentré aqueux sur une membrane tubulaire appropriée, soit une ultrafiltration suivie d'une extraction du filtrat par un mélange d'ester tel que l'acétate d'éthyle ou par un mélange d'éther avec un hydrocarbure aromatique tel que le toluène, après concentration par osmose inverse. Ce procédé demande un outillage compliqué, donc coûteux.

Par ailleurs, les extraits peuvent contenir des produits phytosanitaires. En effet, actuellement, le monde agricole, dans le souci d'assurer une bonne productivité, a recours à une large gamme de produits chimiques pour le traitement et la prévention de diverses maladies des plantes. Bien que l'utilisation de ces produits soit soumise à une règlementation stricte à cause du danger qu'ils représentent sur le plan sanitaire pour l'homme, aucune source végétale n'est épargnée. Il peut donc se produire, lors du processus d'extraction, un enrichissement de l'extrait en produits phytosanitaires qui est inacceptable dans les produits à usage pharmaceutique, cosmétique ou alimentaire. Aucun des documents mentionnés ci-dessus ne décrit le problème posé par les produits phytosaniaires.

La présente invention concerne un procédé qui permet d'éviter les inconvénients ci-dessus et d'obtenir un extrait ayant une faible teneur, voire une teneur nulle en phytosanitaires et ayant un taux de monomères contrôlé.

Selon ce procédé, on traite un extrait végétal aqueux par un solvant des polyphénols tel que l'acétate d'éthyle puis par un solvant chloré tel que chloroforme et le chlorure de méthylène.

La présente invention a, par conséquent, pour objet un procédé de préparation d'extraits contenant des composés polyphénoliques oligomères de type cathéchiques, à partir de sources végétales variées, caractérisé par les étapes suivantes :
a) on extrait le végétal par l'eau chaude ;
b) on précipite les tanins de la phase aqueuse obtenue par addition d'un sel minéral et on filtre les tanins ;
c) on traite le filtrat salé obtenu par l'acétate d'éthyle ;
d) on concentre la phase organique obtenue sous pression réduite ;
e) on ajoute de l'eau distillée ;
f) on élimine l'acétate d'éthyle par évaporation sous pression réduite ;
g) on lave au moins une fois la phase aqueuse obtenue à l'aide d'un solvant chloré ;
h) on élimine le solvant chloré résiduel de la phase aqueuse lavée, et
i) on sèche le concentré aqueux obtenu pour obtenir un extrait.

Selon l'invention, à l'étape a), l'extraction à l'eau est effectuée à une température comprise entre environ 75 et 85°C pendant un temps très court, généralement d'environ 20 à 40 minutes. Il est possible d'utiliser une eau de forage ou une eau de ville sans traitement supplémentaire, à part une éventuelle filtration. Le végétal utilisé peut être broyé ou non.

Le sel utilisé pour précipiter les tanins à l'étape b) est, de préférence, du chlorure de sodium. La précipitation des tanins est avantageusement effectuée à une température comprise en particulier entre 60 et 80°C. La filtration des tanins après précipitation est avantageusement effectuée sous pression et à température ambiante.

L'acétate d'éthyle utilisé à l'étape c) pour traiter la phase aqueuse obtenue après filtration des tanins, un solvant non-miscible à l'eau, est susceptible de dissoudre préférentiellement les polyphénols.

La phase organique est reprise à l'eau distillée à l'étape e) et la solution obtenue est concentrée sous pression réduite à l'étape f), de préférence à une température comprise entre la température ambiante et 50°C, de façon à éliminer la totalité de l'acétate d'éthyle et à obtenir un concentré aqueux.

Le concentré aqueux est lavé à l'étape g) au moins une fois par un solvant chloré non-miscible à l'eau qui est, en particulier, susceptible de dissoudre les produits phytosanitaires. Il est évident que ce solvant chloré doit être exempt de produits sanitaires. Le solvant chloré est, avantageusement, le chloroforme ou le chlorure de méthylène. On élimine ensuite le solvant chloré résiduel du concentré aqueux, avantageusement par évaporation sous pression réduite à une température ne dépassant pas 50°C.

A l'étape i), on sèche l'extrait obtenu par tout procédé connu, en particulier par nébulisation ou par lyophilisation.

L'extrait obtenu après cette étape est prêt à l'usage et peut être directement commercialisé.

Cependant, lorsqu'une purification supplémentaire est nécessaire, en particulier lorsqu'on désire réduire encore le taux de monomères, on effectue un traitement supplémentaire de l'extrait.

Selon un premier mode de réalisation, on effectue une précipitation. L'extrait est dissous dans une quantité minimale d'un solvant organique dissolvant les polyphénols : l'acétate d'éthyle. La solution obtenue, éventuellement filtrée, est ajoutée dans un grand volume de solvant chloré qui ne dissout pas les polyphénols : le chloroforme ou le chlorure de méthylène, de façon à précipiter les polyphénols. L'opération peut se faire à chaud sans dépasser 50°C ou à température ambiante. Le précipité est filtré et séché à une température ne dépassant pas 40°C pour obtenir un extrait purifié ayant une faible teneur en monomères.

Selon un second mode de réalisation, on effectue une extraction solide/liquide. On traite, en continu ou en discontinu, l'extrait à l'aide d'un mélange de deux solvants organiques, l'un des solvants dissolvant les polyphénols : l'acétate d'éthyle et l'autre ne les dissolvant pas : solvant chloré, en particulier le chloroforme ou le chlorure de méthylène. Les proportions entre les deux solvants sont fonction du taux de monomères toléré. Le produit insoluble obtenu est séché à une température ne dépassant pas 40°C. On obtient un extrait purifié ayant une teneur contrôlée en monomères.

La présente invention a également pour objet les extraits contenant des composés polyphénoliques oligomères de type catéchique ayant une teneur contrôlée en monomères phénoliques et une faible teneur en produits phytosanitaires obtenus par le procédé décrit ci-dessus.

Pour mieux présenter l'invention, on va en décrire, à titre purement illustratif et non limitatif, la mise en oeuvre à travers l'exemple suivant :

### EXEMPLE

1 kg de pépins de raisin secs et non broyés sont mis en suspension dans 5 litres d'eau contenant du bisulfite de sodium. Le mélange est porté à 80°C à l'abri de la lumière. Cette température est maintenue pendant 30 minutes. Le mélange est filtré à chaud. Le filtrat chaud est saturé en chlorure de sodium et laissé revenir à température ambiante.

On filtre le précipité abondant de tanins. Le filtrat limpide est épuisé par l'acétate d'éthyle (volume égal à 1/3 de la phase aqueuse). La phase organique est concentrée sous pression réduite sans dépasser 40°C jusqu'à un taux de matière sèche de 20 %. Sur le concentré, on ajoute de l'eau distillée (deux fois le volume). Le mélange est concentré sous vide jusqu'à élimination totale de l'acétate d'éthyle. La phase aqueuse est lavée trois fois par du chloroforme exempt de pesticides. La phase aqueuse lavée est concentrée sous vide pour éliminer le chloroforme jusqu'à un taux de matière sèche de 30 %. Le concentré obtenu est séché par nébulisation.

Le nébulisat obtenu est mis en suspension dans un mélange acétate d'éthyle-chloroforme (7:3) maintenu à 50°C sous agitation pendant une heure. Le mélange encore chaud est filtré sous pression et le solide séché. Le filtrat ne contient que de la (+)catéchine et de la (-)épicatéchine, comme le montre la chromatographie sur couche mince de silice après peracétylation du résidu d'évaporation du solvant chloré.

## Revendications

1. Procédé de préparation d'un extrait contenant des composés polyphénoliques oligomères de type catéchique, à partir de sources végétales variées, caractérisé par les étapes suivantes :
a) on extrait le végétal par l'eau chaude ;
b) on précipite les tanins de la phase aqueuse obtenue par addition d'un sel minéral et on filtre les tanins ;
c) on traite le filtrat salé obtenu par l'acétate d'éthyle ;
d) on concentre la phase organique obtenue sous pression réduite ;
e) on ajoute de l'eau distillée ;
f) on élimine l'acétate d'éthyle par évaporation sous pression réduite ;
g) on lave au moins une fois la phase aqueuse obtenue à l'aide d'un solvant chloré ;
h) on élimine le solvant chloré résiduel de la phase aqueuse lavée, et
i) on sèche le concentré aqueux obtenu pour obtenir un extrait.

2. Procédé selon la revendication 1, caractérisé par le fait qu'à l'étape a) l'extraction à l'eau chaude est effectuée à une température comprise entre environ 75 et 85°C pendant 20 à 40 minutes environ.

3. Procédé selon l'une des revendications 1 ou 2, caractérisé par le fait qu'à l'étape a) on utilise une eau de forage ou une eau de ville sans traitement supplémentaire.

4. Procédé selon l'une des revendications 1 à 3, caractérisé par le fait qu'à l'étape b) on précipite les tanins à l'aide de chlorure de sodium.

5. Procédé selon la revendication 4, caractérisé par le fait qu'on effectue la précipitation à une température comprise entre 60 et 80°C environ.

6. Procédé selon l'une des revendications 1 à 5, caractérisé par le fait qu'à l'étape b), on effectue la filtration des tanins sous pression et à température ambiante.

7. Procédé selon l'une des revendications 1 à 6, caractérisé par le fait qu'à l'étape f), on élimine l'acétate d'éthyle par évaporation sous pression réduite à une température comprise entre la température ambiante et 50°C.

8. Procédé selon l'une des revendications 1 à 7, caractérisé par le fait qu'on choisit le solvant chloré utilisé à l'étape g) dans le groupe formé par le chloroforme et le chlorure de méthylène.

9. Procédé selon l'une des revendications 1 à 8, caractérisé par le fait qu'à l'étape h), on élimine le solvant chloré par évaporation sous pression réduite à une température comprise entre la température ambiante et 50°C.

10. Procédé selon l'une des revendications 1 à 9, caractérisé par le fait qu'à l'étape i), on sèche le concentré aqueux obtenu par nébulisation ou lyophilisation.

11. Procédé selon l'une des revendications 1 à 10, caractérisé par le fait que l'on dissout l'extrait obtenu à l'étape i) dans l'acétate d'éthyle et que l'on ajoute à la solution obtenue un solvant chloré de façon à obtenir un précipité, ledit précipité étant filtré et séché pour obtenir un extrait purifié.

12. Procédé selon l'une des revendications 1 à 10, caractérisé par le fait que l'on traite l'extrait obtenu à l'étape i) à l'aide d'un mélange d'acétate d'éthyle et de solvant chloré et on sèche le produit obtenu pour obtenir un extrait purifié.

13. Procédé selon l'une des revendications 11 ou 12, caractérisé par le fait que le solvant chloré est le chloroforme ou le chlorure de méthylène.

14. Extrait contenant des composés polyphénoliques oligomères de type catéchique susceptible d'être obtenu par le procédé selon l'une des revendications 1 à 13.

## Patentansprüche

1. Verfahren zur Gewinnung eines catechinische oligomere Polyphenolverbindungen aufweisenden Extraktes aus verschiedenen pflanzlichen Quellen, gekennzeichnet durch die folgenden Verfahrensschritte:
a) Extrahieren der Pflanze mit Heißwasser;
b) Ausfällen von Tannin aus der wäßrigen Phase, welche durch Zugabe von Mineralsalz erhalten wird, und filtern des Tannin;
c) Behandeln des erhaltenen, unreinen Filtrats mit Ethylacetat;
d) Anreichern der erhaltenen organischen Phase bei vermindertem Druck;
e) Hinzufügen von destilliertem Wasser;
f) Abtrennen des Ethylacetats durch Verdampfen bei vermindertem Druck;
g) mindestens einmaliges Waschen der durch ein chlorhaltiges Lösungsmittel erhaltenen wäßrigen Phase;
h) Abtrennen des in der gewaschenen wäßrigen Phase verbleibenden chlorhaltigen Lösungsmittels; und
i) Trocknen des erhaltenen wäßrigen Konzentrats, um das Extrakt zu gewinnen.

2. Verfahren nach Anspruch 1, dadurch gekennzeichnet, daß im Schritt a die Extraktion mit Heißwasser bei einer Temperatur von ungefähr 75 bis 85°C in einem Zeitinterval von ungefähr 20 bis 40 Minuten durchgeführt wird.

3. Verfahren nach Anspruch 1 oder 2, dadurch gekennzeichnet, daß im Schritt a Bohrwasser oder Leitungswasser ohne zusätzliche Behandlung verwendet wird.

4. Verfahren nach einem der Ansprüche 1 bis 3, dadurch gekennzeichnet, daß im Schritt b das Tannin mittels Natriumchlorid ausgefällt wird.

5. Verfahren nach Anspruch 4, dadurch gekennzeichnet, daß die Ausfällung bei einer Temperatur von ungefähr 60 bis 80°C durchgeführt wird.

6. Verfahren nach einem der Ansprüche 1 bis 5, dadurch gekennzeichnet, daß im Schritt b die Filtration des Tannin bei Umgebungsdruck und Umgebungstemperatur durchgeführt wird.

7. Verfahren nach einem der Ansprüche 1 bis 6, dadurch gekennzeichnet, daß im Schritt f das Verdampfen des Ethylacetats bei vermindertem Druck und bei einer Temperatur zwischen Raumtemperatur und 50°C durchgeführt wird.

8. Verfahren nach einem der Ansprüche 1 bis 7, dadurch gekennzeichnet, daß das im Schritt g verwendete chlorhaltige Lösungsmittel aus einer Gruppe ausgewählt wird, welche Chloroform und Methylenchlorid aufweist.

9. Verfahren nach einem der Ansprüche 1 bis 8, dadurch gekennzeichnet, daß im Schritt h das Verdampfen des chlorhaltigen Lösungsmittels bei vermindertem Druck und bei einer Temperatur zwischen Raumtemperatur und 50°C durchgeführt wird.

10. Verfahren nach einem der Ansprüche 1 bis 9, dadurch gekennzeichnet, daß im Schritt i das erhaltene wäßrige Konzentrat durch Zerstäuben oder Gefriertrocknen gewaschen wird.

11. Verfahren nach einem der Ansprüche 1 bis 10, dadurch gekennzeichnet, daß das im Schritt i erhaltene Extrakt in Ethylacetat gelöst, und der erhaltenen Lösung ein chlorhaltiges Lösungsmittel zugefügt wird, um ein Ausfällungsprodukt zu erhalten, welches gefiltert und gewaschen wird, um ein reines Extrakt zu erzielen.

12. Verfahren nach einem der Ansprüche 1 bis 10, dadurch gekennzeichnet, daß das im Schritt i erhaltene Extrakt mit einem Gemisch aus Ethylacetat und chlorhaltigem Lösungsmittel behandelt und das erhaltene Produkt getrocknet wird, um ein reines Extrakt zu erzielen.

13. Verfahren nach Anspruch 11 oder 12, dadurch gekennzeichnet, daß das chlorhaltige Lösungsmittel Chloroform oder Methylenchlorid aufweist.

14. Extrakt mit catechinischen oligomeren Polyphenolverbindungen, welches durch das Verfahren nach einem der Ansprüche 1 bis 13 erzielbar ist.

## Claims

1. Process for the preparation of an extract containing polyphenolic oligomer compounds of the catechuic type from various plant sources, characterised by the following steps :
a) the plant is extracted using hot water;
b) the tannins of the resulting aqueous phase are precipitated by the addition of a mineral salt and the tannins are filtered off;
c) the salt filtrate obtained is treated with ethyl acetate;
d) the organic phase obtained is concentrated under reduced pressure;
e) distilled water is added;
f) the ethyl acetate is removed by evaporation under reduced pressure;
g) the aqueous phase obtained is washed at least once with a chlorine-containing solvent;
h) the residual chlorine-containing solvent is removed from the washed aqueous phase, and
i) the aqueous concentrate obtained is dried to obtain an extract.

2. Process according to claim 1, characterised in that in Step a) the extraction using hot water is carried out at a temperature of approximately from 75 to 85°C for approximately from 20 to 40 minutes.

3. Process according to either claim 1 or claim 2, characterised in that there is used in Step a) well water or tap water that has not been additionally treated.

4. Process according to any one of claims 1 to 3, characterised in that in Step b) the tannins are precipitated with sodium chloride.

5. Process according to claim 4, characterised in that the precipitation is carried out at a temperature of approximately from 60 to 80°C.

6. Process according to any one of claims 1 to 5, characterised in that in Step b) the filtration of the tannins is carried out under pressure and at room temperature.

7. Process according to any one of claims 1 to 6, characterised in that in Step f) the ethyl acetate is removed by evaporation under reduced pressure at a temperature of from room temperature to 50°C.

8. Process according to any one of claims 1 to 7, characterised in that the chlorine-containing solvent used in Step g) is selected from the group formed by chloroform and methylene chloride.

9. Process according to any one of claims 1 to 8, characterised in that in Step h) the chlorine-containing solvent is removed by evaporation under reduced pressure at a temperature of from room temperature to 50°C.

10. Process according to any one of claims 1 to 9, characterised in that in Step i) the aqueous concentrate obtained is dried by nebulisation or lyophilisation.

11. Process according to any one of claims 1 to 10, characterised in that the extract obtained in Step i) is dissolved in ethyl acetate, and a chlorine-containing solvent is added to the resulting solution so as to obtain a precipitate, the said precipitate being filtered and dried to obtain a purified extract.

12. Process according to any one of claims 1 to 10, characterised in that the extract obtained in Step i) is treated with a mixture of ethyl acetate and chlorine-containing solvent and the product obtained is dried to obtain a purified extract.

13. Process according to either claim 11 or claim 12, characterised in that the chlorine-containing solvent is chloroform or methylene chloride.

14. Extract containing polyphenolic oligomer compounds of the catechuic type that can be obtained by the process according to any one of claims 1 to 13.
